# EUROPÄISCHE PATENTSCHRIFT

(11) **EP 0 333 984 B1**
(45) Veröffentlichungstag und Bekanntmachung des Hinweises auf die Patenterteilung: **07.04.1993**
(21) Anmeldenummer: 89100700.7
(22) Anmeldetag: 17.01.1989
(51) Int. Cl.: C07C 43/13, C07C 41/03, C07C 41/26, C07C 41/42

(54) **Verfahren zur Herstellung von Polyglycerinen**
Process for the preparation of polyglycerins
Procédé de préparation de polyglycérines

(30) Priorität: 24.03.1988 DE 3809882
(43) Veröffentlichungstag der Anmeldung: 27.09.1989
(73) Patentinhaber: DEUTSCHE SOLVAY-WERKE GMBH, 42697 Solingen (DE)
(72) Erfinder: Jakobson, Gerald, Dr. Dipl.-Chem., D-4134 Rheinberg 2 (DE); Siemanowski, Werner, Dr. Dipl.-Chem., D-4134 Rheinberg 1 (DE)
(74) Vertreter: Seiler, Siegfried

(56) Entgegenhaltungen:
- DE-A- 3 410 520
- DE-C- 510 422
- US-A- 2 520 670
- US-A- 4 053 525

## Beschreibung

Die vorliegende Erfindung betrifft ein Verfahren zur Herstellung von Polyglycerinen, die arm an cyclischen Komponenten sind, durch Umsetzung von Glycerin mit Chlorhydrinen bei bestimmten Temperaturen und die Auftrennung des Reaktionsgemisches.

Nach der DE-C-510 422 werden Oxyalkyläther des Glycerins dadurch hergestellt, daß man Alkylenoxyde auf Glycerin bei erhöhten Temperaturen einwirken läßt. Im Beispiel 5 dieser Druckschrift wird Epichlorhydrin mit Glycerin gekocht und bis auf 200 °C erhitzt, wobei das entstehende Gemisch, das γ-Chlor-ß-oxypropyläther des Glycerins und darüber hinaus in erheblichem Umfang andere Polymerisate enthält, die bei einer beabsichtigten Weiterverarbeitung das Verfahren stören.

Gemäß Literaturstelle US-A-4 053 525 werden Chlorhydrin enthaltende Reaktionsmischungen, die z.B. Epichlorhydrin und Glycerinmono- und -dichlorhydrin enthalten, bei Temperaturen von 50 - 70 °C in Gegenwart einer wenigstens 0,5 Gew.-%igen Alkalicarbonatlösung und organischen Lösemitteln in einem Zeitraum von 20 Std. bis 150 Std. umgesetzt.

Das Verfahren gemäß US-A-4 053 525 läßt sich daher nur im Labormaßstab durchführen und ist für großtechnische Produktionen im Verbund mit anderen Verfahrensstufen nicht geeignet.

Bei der Patentanmeldung DE-A-34 10 520 wird von einem Destillationsrückstand aus der synthetischen Glycerinherstellung über Epichlorhydrin ausgegangen. Bei diesem Verfahren werden als Hauptprodukt nicht Polyglycerin, sondern das Glycerin (Monoglycerin) hergestellt. Der bei der Destillation des Monoglycerins (das das Hauptprodukt darstellt) anfallende Destillationsrückstand stellt eine zähe, bei Umgebungstemperatur feste bis breiige Masse mit einem sehr hohen Salzgehalt dar und wird volkstümlich wegen seiner Zähigkeit und dunklen Verfärbung auch Glycerinpech genannt. Anschließend wird der Salzgehalt durch die Wasserverdünnung auf 5 - 30 Gew.-% eingestellt.

Ein Verfahren zur Herstellung von Polyglycerinen ist aus der US-PS 25 20 670 bekannt, bei dem Glycerin mit Glycerin-α-Monochlorhydrin in Gegenwart von konzentriertem Alkali bei erhöhter Temperatur zu einem Gemisch von Polyglycerinen umgesetzt wird. Dieses Verfahren hat den Nachteil der relativ langen Reaktionsdauer und daß nach Beendigung der Reaktion das Reaktionsgemisch mit niederen aliphatischen Alkoholen aufgearbeitet werden muß.

Angaben über die erzielten Ausbeuten an Polyglycerinen sowie über deren Gehalt an cyclischen Komponenten werden nicht gemacht.

Aufgabe der vorliegenden Erfindung war es daher, ein Verfahren zu finden, mit dessen Hilfe Polyglycerine in guten Ausbeuten und mit nur geringem Anteil an cyclischen Komponenten erhalten werden. Hierbei sollte gleichzeitig eine Isolierung der Zwischenprodukte (Chlorhydrinethermischung) nicht erforderlich sein sowie aus Umweltschutzgründen eine Aufarbeitung der Endprodukte durch Behandlung mit organischen Lösungsmitteln vermieden werden. Das Verfahren sollte großtechnisch durchführbar sein.

Erfindungsgemäß wurde festgestellt, daß diese Aufgabe dadurch gelöst wird, daß Glycerin oder Diglycerin oder ein höheres Polyglycerin mit Epichlorhydrin (anstelle von Chlorhydrin) bei Temperaturen von 90 bis 170 °C, in einem Molverhältnis von Glycerin oder Diglycerin oder höherem Polyglycerin zu Epichlorhydrin von 5 : 1 bis 1 : 3 umgesetzt und dem erhaltenen, nicht aufgetrennten Reaktionsgemisch bei einer Temperatur von 50°C bis 120°C, vorzugsweise von 80 °C bis 95 °C, entsprechend dem Gehalt des Reaktionsgemisches an organisch gebundenem Chlor ein alkalisch reagierendes Medium zugegeben wird, das Reaktionsgemisch durch Wasserzugabe auf eine 70 - 40gew.-%ige Lösung verdünnt und bei Temperaturen von 30 °C bis 80 °C über eine Kombination von stark sauren Kationen- und nachfolgenden schwach basischen Anionenaustauschern entsalzt, durch Destillation entwässert und das Glycerin-Polyglyceringemisch durch fraktionierte Destillation in Glycerin, Diglycerin und höhere Polyglycerine aufgetrennt wird.

Nach einer bevorzugten Ausführungsform wird das Glycerin oder Diglycerin oder ein höheres Polyglycerin mit Epichlorhydrin bei Temperaturen von 120 °C bis 150 °C in einem Molverhältnis von Glycerin oder Diglycerin oder höherem Polyglycerin zu Epichlorhydrin von 5 : 1 bis 1 : 3 umgesetzt und dem erhaltenen, nicht aufgetrennten Reaktionsgemisch bei einer Temperatur von 80 °C bis 95 °C, entsprechend dem Gehalt des Reaktionsgemisches an organisch gebundenem Chlor eine alkalisch reagierende äßrige Lösung zugegeben, das Reaktionsgemisch durch Wasserzugabe auf eine 60 - 50gew.%ige Lösung verdünnt und bei Temperaturen von 40 °C bis 60 °C über eine Kombination von stark sauren Kationen- und nachfolgenden schwach basischen Anionenaustauschern entsalzt und nachfolgend durch Destillation entwässert und durch fraktionierte Destillation aufgetrennt.

Nach einer bevorzugten Ausführungsform wird dem nicht aufgetrennten Reaktionsgemisch aus der Umsetzung von Epichlorhydrin mit Glycerin, Diglycerin oder einem höheren Polyglycerin eine alkalisch reagierende Alkalicarbonatlösung, vorzugsweise eine konzentrierte Sodalösung, zugegeben.

Vorteilhaft wird die alkalisch reagierende Alkalicarbonatlösung dem nicht aufgetrennten Reaktionsgemisch aus der Umsetzung von Epichlorhydrin mit Glycerin, Diglycerin oder einem höheren Polyglycerin im äquivalenten Verhältnis von Alkalicarbonat zu dem Gehalt an organisch gebundenem Chlor von 1 : 1 bis 1,2 : 1, vorzugsweise 1,05 : 1 bis 1,1 : 1, zugegeben.

Zweckmäßig wird das Reaktionsgemisch durch die Zugabe der alkalisch reagierenden wäßrigen Lösung auf einen pH-Bereich von 7,0 bis 11,5, vorzugsweise 8 bis 11, eingestellt.

Nach einer weiteren Ausführungsform wird das Reaktionsgemisch auf Raumtemperatur abgekühlt und die Hauptmenge des ausgefällten Salzes abgetrennt, vorzugsweise abfiltriert.

Durch Variation der Molverhältnisse von Glycerin zu Epichlorhydrin sowie durch Einsatz von Diglycerin oder höheren Polyglycerinen anstelle von Glycerin läßt sich erfindungsgemäß die gewünschte Zusammensetzung der Polyglycerinmischung je nach Bedarf variieren.

So beträgt nach einer Ausführungsform das Molverhältnis von Glycerin zu Epichlorhydrin zur Erhöhung des Diglycerinanteiles 4 : 1 bis 2,5 : 1.

Nach einer weiteren Ausführungsform beträgt das Molverhältnis von Glycerin und/oder Diglycerin zu Epichlorhydrin zur Erhöhung des neben Diglycerin anfallenden Polyglycerinanteiles 2,49 : 1 bis 0,5 : 1, vorzugsweise 1,8 : 1 bis 0,4 : 1.

Nach einer anderen Ausführungsform wird die alkalisch reagierende Alkalicarbonatlösung dem nicht aufgetrennten Reaktionsgemisch aus der Umsetzung von Epichlorhydrin mit Glycerin, Diglycerin oder einem höheren Polyglycerin in geringem Überschuß zugegeben und nach beendeter Reaktion dieser Überschuß neutralisiert.

Zur Neutralisation wird bevorzugt Salzsäure eingesetzt, jedoch können auch andere Mineralsäuren oder saure Kationenaustauscher verwendet werden.

Erfindungsgemäß wurde weiterhin festgestellt, daß mit der Änderung der Molverhältnisse eine Änderung der Reaktionszeit einhergeht. Es ergab sich, daß hinsichtlich der Reaktionszeit und des gewünschten minimalen Gehalts der Roh-Polyglycerinmischung an cyclischen Komponenten ein Molverhältnis von Glycerin und/oder Diglycerin zu Epichlorhydrin von 2,5 bis 1,0 : 1 sich als besonders günstig erwies.

Die Regenerierung der Kationenaustauschermasse in den Kationenaustauschern erfolgt bevorzugt mittels einer Gleichstrom- oder Verbund-Gleichstrom-Regenerierung.

Nach einer bevorzugten Ausführungsform werden nach der Regenerierung die Salze ausgewaschen. Nach Beeindigung des Auswaschvorganges wird die polyglycerinhaltige, vorzugsweise diglycerinhaltige Lösung durch die Ionenaustauscher durchgeleitet und die den Anionenaustauscher verlassende polyglycerinhaltige, vorzugsweise diglycerinhaltige Lösung bis zur Erreichung eines Polyglyceringehaltes, vorzugsweise Diglyceringehaltes, von 20 Gew.-%, vorzugsweise bis zur Erreichung eines Polyglyceringehaltes, vorzugsweise Diglyceringehaltes, von 15 Gew.-%, zurückgeleitet und zur Herstellung der 70 - 40 gew.-%igen, vorzugsweise 60 - 50 gew.-%igen, polyglycerinhaltigen, vorzugsweise diglycerinhaltigen Ausgangslösung mitverwendet.

Die Durchleitung der polyglycerinhaltigen, vorzugsweise diglycerinhaltigen Lösung erfolgt durch die Ionenaustauscher bevorzugt unter einem Überdruck.

Die polyglycerinhaltige, vorzugsweise diglycerinhaltige Lösung wird dabei unter einem Druck von 1,1 - 10 bar, vorzugsweise 2 - 6 bar, durch die Ionenaustauscher, d. h. durch einen oder mehreren Kationenaustauscher und mindestens einen Anionenaustauscher, geleitet. Zur Steuerung und Aufrechterhaltung des Druckes sind an einer oder mehreren Stellen in der Leitung oder an den Ionenaustauschern Ventile angebracht.

Dabei wird zweckmäßig die polyglycerinhaltige, vorzugsweise diglycerinhaltige Lösung mit einer Strömungsgeschwindigkeit von 0,5 m/h bis 15 m/h, vorzugsweise 1m/h bis 5m/h, durch die Ionenaustauscher geleitet.

Als Kationenaustauschermassen und Anionenaustauschermassen werden bevorzugt solche verwendet, die temperaturbeständig bis über 80 °C, vorzugsweise bis über 100°C, sind.

Die Ionenaustauschermasse des Kationenaustauschers und/oder Anionenaustauschers ist zweckmäßig von einer Siebplatte, Lochplatte oder einer in der Höhenrichtung des Ionenaustauschers verschiebbar angeordneten, die Austauschermasse abdeckenden und einen gleichmäßigen Flüssigkeitsdurchtritt ermöglichenden Vorrichtung und/oder einer inerten Preßmasse und/oder elastischen Kunststoffmasse bedeckt.

Die stark saure Kationenaustauschermasse und die schwach basische Anionenaustauschermasse besitzen bevorzugt eine innere Oberfläche (gemessen nach Methode BET) von mehr als 25 m²/g, vorzugsweise 50 bis 100 m²/g.

### Ausführungsbeispiele:

### 1. Ausführungsbeispiel für die Herstellung von Polyglycerin mit einem höheren Anteil an Diglycerin:

1,474 kg (16 mol) Glycerin und 740 g (8 mol) Epichlorhydrin wurden in einen 2-l-Doppelwandreaktor (Heizflüssigkeit: Öl) gegeben. Unter Rühren wurde das 2-phasige Reaktionsgemisch bis zum Sieden (Rückfluß) erhitzt. Nach ca. 80 min wurde die Ölvorlauftemperatur auf ca. 145 °C erhöht, wobei noch nicht abreagiertes Epichlorhydrin wieder unter Rückfluß siedete. Nach 50 min bei 145 °C Ölvorlauftemperatur wurde diese auf 140 °C gedrosselt. Somit ließ sich die schwach exotherme Reaktion abfangen und die Reaktionstemperatur bei 150 °C halten. Nach ca. 50 min hatten sich die Ölvorlauftemperatur und die Temperatur der Reaktionslösung angeglichen. Nach insgesamt 3,25 h war die Umsetzung beendet.
- Rohauswaage:: 2,205 kg - 1,75 l

1,284 kg ( = organisch gebundenes Chlor : 4,776 mol) dieser rohen Chlorhydrinethermischung wurden in dem gleichen Reaktor vorgelegt und auf 90°C erwärmt. Innerhalb von 1 h wurden 1,125 l (entsprechend dem Gehalt an organisch gebundenem Chlor plus 5 % Überschuß) einer 2,23 molaren Sodalösung bei 90 °C zudosiert (moderate CO₂-Entwicklung). Nach weiteren 1,5 h bei dieser Temperatur wurde die Heizung abgestellt und der Reaktionsansatz durch Zugabe von 1/2 konz. Salzsäure neutralisiert.
- Rohauswaage:: 2,516 kg
- Endvolumen :: ca. 2,15 l

Die neutrale Reaktionslösung wurde mit Wasser verdünnt, über eine Kombination von stark sauren Kationen- und schwach basischem Anionenaustauscher entsalzt und das Wasser im Vakuum abgedampft.
- Endauswaage:: 1.041 kg

Das Produktgemisch hatte folgende Zusammensetzung in Gew.-%: Glycerin 43,5, cyclisches Diglycerin 0,9, Diglycerin 38,5, cyclisches Triglycerin 0,4, Triglycerin 11,8, cyclisches Tetraglycerin 0,1, Tetraglycerin 3,0, Pentaglycerin 0,7, Hexaglycerin 0,1.

Erhaltene Werte des Polyglyceringemisches in Gew.-% bei Änderung des Molverhältnisses Glycerin/Epichlorhydrin 3 : 1, 2 : 1, 1 : 1, und 1 : 2

| | 1 : 1 | 2 : 1 | 3 : 1 | 1 : 2 |
|---|---|---|---|---|
| Glycerin | 25,4 | 43,5 | 53,6 | 37,2 |
| cyclisches Diglycerin | 1,5 | 0,9 | 1,2 | 1,5 |
| Diglycerin | 42,4 | 38,5 | 33,1 | 41,7 |
| cyclisches Triglycerin | 1,0 | 0,4 | - | 0,8 |
| Triglycerin | 19,6 | 11,8 | 8,4 | 13,6 |
| cyclisches Tetraglycerin | 0,9 | 0,1 | 0,7 | 0,5 |
| Tetraglycerin | 5,7 | 3,0 | 1,8 | 3,1 |
| Pentaglycerin | 2,4 | 0,7 | 0,6 | 1,0 |
| Hexaglycerin | 0,6 | 0,1 | - | - |

### 2. Ausführungsbeispiel für die erfindungsgemäße Herstellung von Polyglycerin mit einem höheren Anteil an Triglycerin:

664,6 g (4 mol) Diglycerin und 185 g (2 mol) Epichlorhydrin wurden in einen 1-l-Doppelwandreaktor (Heizflüssigkeit: Öl) gegeben. Unter Rühren wurde das 2-phasige Reaktionsgemisch bis zum Sieden (Rückfluß, Badtemperatur 130 °C) erhitzt. Nach ca. 1 h wurde die Ölvorlauftemperatur auf 145 °C erhöht und für ca. 2 h bei dieser Temperatur belassen.

Nach insgesamt ca. 3 h war die Umsetzung beendet.
- Rohauswaage:: 849 g - 0,7 l

784,9 g (= organisch gebundenes Chlor: 1,848 mol) dieser rohen Chlorhydrinethermischung wurden in einem 2-l-Doppelwandreaktor vorgelegt und auf 90 °C erwärmt. Innerhalb von ca. 45 min wurden 485 ml einer 2 molaren Sodalösung bei 90 °C zudosiert (moderate CO₂-Entwicklung). Nach weiteren 1,5 h bei dieser Temperatur wurde die Heizung abgestellt und der Reaktionsansatz durch Zugabe von 1/2 konz. Salzsäure neutralisiert.
- Rohauswaage:: 1,315 kg - 1,15 l

Die neutrale Reaktionslösung wurde mit Wasser verdünnt, über eine Kombination von stark sauren Kationen- und schwach basischen Anionenaustauschern entsalzt und das Wasser im Vakuum abgedampft.
- Endauswaage:: 715 g

Das Produktgemisch hatte folgende Zusammensetzung in Gew.-%:
Glycerin 4,9, cyclisches Diglycerin 0,1, Diglycerin 50,8, cyclisches Triglycerin 2,1, Triglycerin 25,6, cyclisches Tetraglycerin 0,5, Tetraglycerin 4,9, Pentaglycerin 6,0, Hexaglycerin 3,5, Heptaglycerin 0,8, Octaglycerin 0,1.

## Patentansprüche

1. Verfahren zur Herstellung von Polyglycerinen, die arm an cyclischen Komponenten sind, durch Umsetzung von Glycerin mit Chlorhydrinen bei erhöhter Temperatur, dadurch gekennzeichnet, daß Glycerin oder Diglycerin oder ein höheres Polyglycerin mit Epichlorhydrin (anstelle von Chlorhydrin) bei Temperaturen von 90 bis 170 °C in einem Molverhältnis von Glycerin oder Diglycerin oder höherem Polyglycerin zu Epichlorhydrin von 5 : 1 bis 1 : 3 umgesetzt und dem erhaltenen, nicht aufgetrennten Reaktionsgemisch bei einer Temperatur von 80 °C bis 120°C entsprechend dem Gehalt des Reaktionsgemisches an organisch gebundenem Chlor ein alkalisch reagierendes Medium zugegeben wird, das Reaktionsgemisch durch Wasserzugabe auf eine 70 - 40gew.-%ige Lösung verdünnt und bei Temperaturen von 30 °C bis 80 °C über eine Kombination von stark sauren Kationen- und nachfolgenden schwach basischen Anionenaustauschern entsalzt, durch Destillation entwässert und das Glycerin-Polyglyceringemisch durch fraktionierte Destillation in Glycerin, Diglycerin und höhere Polyglycerine aufgetrennt wird.

2. Verfahren nach Anspruch 1, dadurch gekennzeichnet, daß Glycerin oder Diglycerin oder ein höheres Polyglycerin mit Epichlorhydrin bei Temperaturen von 120 °C bis 150 °C in einem Molverhältnis von Glycerin oder Diglycerin oder höherem Polyglycerin zu Epichlorhydrin von 5 : 1 bis 1 : 3 umgesetzt und dem erhaltenen, nicht aufgetrennten Reaktionsgemisch bei einer Temperatur von 80 °C bis 95 °C, entsprechend dem Gehalt des Reaktionsgemisches an organisch gebundenem Chlor eine alkalisch reagierende wäßrige Lösung zugegeben wird, das Reaktionsgemisch durch Wasserzugabe auf eine 60 - 50gew.%ige Lösung verdünnt und bei Temperaturen von 40 °C bis 60°C über eine Kombination von stark sauren Kationen- und nachfolgenden schwach basischen Anionenaustauschern entsalzt wird und nachfolgend durch Destillation entwässert und durch fraktionierte Destillation aufgetrennt wird.

3. Verfahren nach Anspruch 1 oder 2, dadurch gekennzeichnet, daß dem nicht aufgetrennten Reaktionsgemisch aus der Umsetzung von Epichlorhydrin mit Glycerin, Diglycerin oder einem höheren Polyglycerin eine alkalisch reagierende Alkalicarbonatlösung, vorzugsweise eine konzentrierte Sodalösung, zugegeben wird.

4. Verfahren nach einem oder mehreren der Ansprüche 1 bis 3, dadurch gekennzeichnet, daß die alkalisch reagierende Alkalicarbonatlösung dem nicht aufgetrennten Reaktionsgemisch aus der Umsetzung von Epichlorhydrin mit Glycerin, Diglycerin oder einem höheren Polyglycerin im äquivalenten Verhältnis von Alkalicarbonat zu dem Gehalt an organisch gebundenem Chlor von
1 : 1 bis 1,2 : 1, vorzugsweise
1,05 : 1 bis 1,1 : 1,
zugegeben wird.

5. Verfahren nach einem oder mehreren der Ansprüche 1 bis 4, dadurch gekennzeichnet, daß das Reaktionsgemisch durch die Zugabe der akalisch reagierenden wäßrigen Lösung auf einen pH-Bereich von
7,0 bis 11,5, vorzugsweise
8 bis 11,
eingestellt wird.

6. Verfahren nach einem oder mehreren der Ansprüche 1 bis 5, dadurch gekennzeichnet, daß das Reaktionsgemisch auf Raumtemperatur abgekühlt und die Hauptmenge des ausgefällten Salzes abgetrennt, vorzugsweise abfiltriert wird.

7. Verfahren nach einem oder mehreren der Ansprüche 1 bis 6, dadurch gekennzeichnet, daß das Molverhältnis von Glycerin zu Epichlorhydrin zur Erhöhung des Diglycerinanteiles,
4 : 1 bis 2,5 : 1
beträgt.

8. Verfahren nach einem oder mehreren der Ansprüche 1 bis 6, dadurch gekennzeichnet, daß das Molverhältnis von Glycerin und/oder Diglycerin zu Epichlorhydrin zur Erhöhung des neben Diglycerin anfallenden Polyglycerinanteiles
2,49 : 1 bis 0,5 : 1, vorzugsweise
1,8 : 1 bis 0,4 : 1,
beträgt.

9. Verfahren nach einem oder mehreren der Ansprüche 1 bis 8, dadurch gekennzeichnet, daß die Regenerierung der Kationenaustauschermasse in den Kationenaustauschern mittels einer Gleichstrom- oder Verbund-Gleichstrom-Regenerierung erfolgt.

10. Verfahren nach einem oder mehreren der Ansprüche 1 bis 8, dadurch gekennzeichnet, daß nach der Regenerierung die Salze ausgewaschen, nach Beendigung des Auswaschvorganges die polyglycerinhaltige, vorzugsweise diglycerinhaltige Lösung durch die Ionenaustauscher durchgeleitet wird und die den Anionenaustauscher verlassende polyglycerinhaltige, vorzugsweise diglycerinhaltige Lösung bis zur Erreichung eines Polyglyceringehaltes, vorzugsweise Diglyceringehaltes, von 20 Gew.-%, vorzugweise bis zur Erreichung eines Polyglyceringehaltes, vorzugsweise Diglyceringehaltes, von 15 Gew.-%, zurückgeleitet und zur Herstellung der
70 - 40 gew.-%igen, vorzugsweise
60 - 50 gew.-%igen,
polyglycerinhaltigen, vorzugsweise diglycerinhaltigen Ausgangslösung mitverwendet wird.

11. Verfahren nach einem oder mehreren der Ansprüche 1 bis 10, dadurch gekennzeichnet, daß die Durchleitung der polyglycerinhaltigen, vorzugsweise diglycerinhaltigen Lösung durch die Ionenaustauscher unter Überdruck erfolgt.

12. Verfahren nach einem oder mehreren der Ansprüche 1 bis 11, dadurch gekennzeichnet, daß die polyglycerinhaltige, vorzugsweise diglycerinhaltige Lösung unter einem Druck von
1,1 - 10 bar, vorzugsweise
2 - 6 bar,
durch einen oder mehrere Kationenaustauscher und mindestens einen Anionenaustauscher geleitet wird.

13. Verfahren nach einem oder mehreren der Ansprüche 1 bis 12, dadurch gekennzeichnet, daß die Ionenaustauschermasse des Kationenaustauschers und/oder Anionenaustauschers von einer Siebplatte, Lochplatte oder einer in der Höhenrichtung des Ionenaustauschers verschiebbar angeordneten, die Austauschermasse abdeckenden und einen gleichmäßigen Flüssigkeitsdurchtritt ermöglichenden Vorrichtung und/oder einer inerten Preßmasse und/oder elastischen Kunststoffmasse bedeckt ist.

14. Verfahren nach einem oder mehreren der Ansprüche 1 bis 13, dadurch gekennzeichnet, daß die eingesetzten Kationenaustauschermassen und Anionenaustauschermassen temperaturbeständig
bis über 80 °C, vorzugsweise
bis über 100 °C,
sind.

15. Verfahren nach einem oder mehreren der Ansprüche 1 bis 14, dadurch gekennzeichnet, daß die stark saure Kationenaustauschermasse und die schwach basische Anionenaustauschermasse eine innere Oberfläche (gemessen nach Methode BET) von
mehr als 25 m²/, vorzugsweise
50 bis 100 m²/g,
aufweisen.

16. Verfahren nach einem oder mehreren der Ansprüche 1 bis 15, dadurch gekennzeichnet, daß die polyglycerinhaltige, vorzugsweise diglycerinhaltige Lösung mit einer Strömungsgeschwindigkeit von
0,5 m/h bis 15 m/h, vorzugsweise
1 m/h bis 5 m/h,
durch die Ionenaustauscher geleitet wird.

## Claims

1. A method for producing polyglycerols which are low in cyclic components, by reacting glycerol with chlorohydrins at elevated temperature, characterised in that glycerol or diglycerol or a higher polyglycerol is reacted with epichlorohydrin (instead of chlorohydrin) at temperatures of 90 to 170°C in a molar ratio of glycerol or diglycerol or higher polyglycerol to epichlorohydrin of 5 : 1 to 1 : 3 and an alkaline-reacting medium is added to the resulting, non-separated reaction mixture at a temperature of 80°C to 120°C corresponding to the content of the reaction mixture in organically bound chlorine, the reaction mixture is diluted to a 70 - 40% by weight solution by the addition of water and desalted at temperatures of 30°C to 80°C over a combination of strongly acidic cation exchangers and subsequent weakly basic anion exchangers, is dehydrated by distillation and the glycerol-polyglycerol mixture is separated by fractional distillation into glycerol, diglycerol and higher polyglycerols.

2. A method according to Claim 1, characterised in that glycerol or diglycerol or a higher polyglycerol is reacted with epichlorohydrin at temperatures of 120°C to 150°C in a molar ratio of glycerol or diglycerol or higher polyglycerol to epichlorohydrin of 5 : 1 to 1 : 3 and an alkaline-reacting aqueous solution is added to the resulting, non-separated reaction mixture at a temperature of 80°C to 95°C, corresponding to the content of the reaction mixture of organically bound chlorine, the reaction mixture is diluted to a 60 - 50% by weight solution by the addition of water and desalted at temperatures of 40°C to 60°C over a combination of strongly acidic cation exchangers and subsequent weakly basic anion exchangers, and is then dehydrated by distillation and separated by fractional distillation.

3. A method according to Claim 1 or 2, characterised in that an alkaline-reacting alkali carbonate solution, preferably a concentrated soda solution, is added to the non-separated reaction mixture from the reaction of epichlorohydrin with glycerol, diglycerol or a higher polyglycerol.

4. A method according to one or more of Claims 1 to 3, characterised in that the alkaline-reacting alkali carbonate solution is added to the non-separated reaction mixture from the reaction of epichlorohydrin with glycerol, diglycerol or a higher polyglycerol in an equivalent ratio of alkali carbonate to the content of organically bound chlorine of
1 : 1 to 1.2 : 1, preferably
1.05 : 1 to 1.1 : 1.

5. A method according to one or more of Claims 1 to 4, characterised in that the reaction mixture is adjusted by the addition of the alkaline-reacting aqueous solution to a pH range of
7.0 to 11.5, preferably
8 to 11.

6. A method according to one or more of Claims 1 to 5, characterised in that the reaction mixture is cooled to room temperature and the major part of the precipitated salt is separated off, preferably filtered off.

7. A method according to one or more of Claims 1 to 6, characterised in that the molar ratio of glycerol to epichlorohydrin is
4 : 1 to 2.5 : 1
in order to increase the diglycerol content.

8. A method according to one or more of Claims 1 to 6, characterised in that the molar ratio of glycerol and/or diglycerol to epichlorohydrin is
2.49 : 1 to 0.5 : 1, preferably
1. 8 : 1 to 0.4 : 1,
in order to increase the content of polyglycerol which is produced in addition to diglycerol.

9. A method according to one or more of Claims 1 to 8, characterised in that the regeneration of the cation exchanger medium in the cation exchangers takes place by means of co-current or compounded co-current regeneration.

10. A method according to one or more of Claims 1 to 8, characterised in that after regeneration the salts are washed out, after the washing process has been completed the polyglycerol-containing, preferably diglycerol-containing, solution is passed through the ion exchanger and the polyglycerol-containing, preferably diglycerol-containing, solution leaving the anion exchanger is conveyed back until a polyglycerol content, preferably diglycerol content, of 20% by weight, is reached, preferably until a polyglycerol content, preferably diglycerol content, of 15% by weight, is reached, and is jointly used to produce the
70 - 40% by weight, preferably
60 - 50% by weight,
polyglycerol-containing, preferably diglycerol-containing, starting solution.

11. A method according to one or more of Claims 1 to 10, characterised in that the conveying of the polyglycerol-containing, preferably diglycerol-containing, solution through the ion exchanger is effected under excess pressure.

12. A method according to one or more of Claims 1 to 11, characterised in that the polyglycerol-containing, preferably diglycerol-containing, solution is conveyed through one or more cation exchangers and at least one anion exchanger under a pressure of
1.1 - 10 bar, preferably
2 - 6 bar.

13. A method according to one or more of Claims 1 to 12, characterised in that the ion exchange medium of the cation exchanger and/or of the anion exchanger is covered by a sieve plate, perforated plate or a device which is arranged to be displaceable in the vertical direction of the ion exchanger, covers the exchanger medium and permits even passage of the liquid and/or by an inert moulding compound and/or elastic plastics compound.

14. A method according to one or more of Claims 1 to 13, characterised in that the cation exchanger media and anion exchange media used are temperature-resistant to
above 80°C, preferably
above 100°C.

15. A method according to one or more of Claims 1 to 14, characterised in that the strongly acidic cation exchanger medium and the weakly basic anion exchanger medium have an internal surface area (measured according to the BET method) of
more than 25 m²/g, preferably
50 to 100 m²/g.

16. A method according to one or more of Claims 1 to 15, characterised in that the polyglycerol-containing, preferably diglycerol-containing, solution is conveyed through the ion exchanger at a flow rate of
0.5 m/h to 15 m/h, preferably
1 m/h to 5 m/h.

## Revendications

1. Procédé pour la préparation de polyglycérines qui sont pauvres en composants cycliques, par la mise en réaction de glycérine avec des chlorhydrines à température élevée, caractérisé en ce qu'on fait réagir de la glycérine ou de la diglycérine ou encore une polyglycérine supérieure avec de l'épichlorhydrine (au lieu de chlorhydrine) à des températures de 90 à 170°C dans un rapport molaire entre la glycérine ou la diglycérine ou encore une polyglycérine supérieure et l'épichlorhydrine de 5 : 1 à 1 : 3 et, au mélange réactionnel obtenu non séparé, à une température de 80°C à 120°C, en fonction de la teneur du mélange réactionnel en chlore lié organiquement, on ajoute un milieu à réaction alcaline, on dilue le mélange réactionnel par addition d'eau pour obtenir une solution à raison de 70 à 40% en poids et on dessale à des températures de 30°C à 80°C par dessus une combinaison d'échangeurs de cations fortement acides et ensuite d'échangeurs d'anions faiblement basiques, on déshydrate par distillation et on sépare le mélange de glycérine-polyglycérines par distillation fractionnée en glycérine, diglycérine et polyglycérines supérieures.

2. Procédé selon la revendication 1, caractérisé en ce qu'on fait réagir de la glycérine ou de la diglycérine ou encore une polyglycérine supérieure avec de l'épichlorhydrine à des températures de 120°C à 150°C dans un rapport molaire entre la glycérine ou la diglycérine ou encore une polyglycérine supérieure et l'épichlorhydrine de 5 : 1 à 1 : 3 et, au mélange réactionnel obtenu non séparé, à une température de 80°C à 95°C, en fonction de la teneur du mélange réactionnel en chlore lié organiquement, on ajoute une solution aqueuse à réaction alcaline, on dilue le mélange réactionnel par addition d'eau pour obtenir une solution à raison de 60 à 50% en poids et on dessale à des températures de 40°C à 60°C par dessus une combinaison d'échangeurs de cations fortement acides et ensuite d'échangeurs d'anions faiblement basiques, après quoi on déshydrate par distillation et on sépare par distillation fractionnée.

3. Procédé selon la revendication 1 ou 2, caractérisé en ce que, au mélange réactionnel non séparé provenant de la mise en réaction de l'épichlorhydrine avec la glycérine, la diglycérine ou une polyglycérine supérieure, on ajoute une solution de carbonate de métaux alcalins à réaction alcaline, de préférence un solution concentrée de carbonate de sodium.

4. Procédé selon une ou plusieurs des revendications 1 à 3, caractérisé en ce qu'on ajoute la solution de carbonate de métaux alcalins à réaction alcaline au mélange réactionnel non séparé provenant de la mise en réaction de l'épichlorhydrine avec la glycérine, la diglycérine ou une polyglycérine supérieure dans un rapport équivalent entre le carbonate de métaux alcalins et la teneur en chlore lié organiquement de
1 : 1 à 1,2 : 1, de préférence de
1,05 : 1 à 1,1 : 1.

5. Procédé selon une ou plusieurs des revendication 1 à 4, caractérisé en ce qu'on règle le mélange réactionnel par addition de la solution aqueuse à réaction alcaline à un domaine de pH de
7,0 à 11,5, de préférence
de 8 à 11.

6. Procédé selon une ou plusieurs des revendication 1 à 5, caractérisé en ce qu'on refroidit le mélange réactionnel à la température ambiante et on sépare la quantité principale du sel précipité, de préférence par filtration.

7. Procédé selon une ou plusieurs des revendication 1 à 6, caractérisé en ce que le rapport molaire entre la glycérine et l'épichlorhydrine, pour augmenter la fraction en diglycérine, s'élève de
4 : 1 à 2,5 : 1.

8. Procédé selon une ou plusieurs des revendication 1 à 6, caractérisé en ce que le rapport molaire entre la glycérine et/ou la diglycérine et l'épichlorhydrine, pour augmenter la fraction en polyglycérines que l'on obtient en plus de la diglycérine, s'élève de
2,49 : 1 à 0,5 : 1, de préférence de
1,8 : 1 à 0,4 : 1.

9. Procédé selon une ou plusieurs des revendication 1 à 8, caractérisé en ce que la régénération de la matière faisant office d'échangeur de cations dans les échangeurs de cations a lieu au moyen d'une régénération en courant continu ou en courant continu-compound.

10. Procédé selon une ou plusieurs des revendication 1 à 8, caractérisé en ce que, suite à la régénération, on élimine les sels par lavage; au terme du processus d'élimination par lavage, on fait passer la solution contenant des polyglycérines, de préférence de la diglycérine, à travers l'échangeur d'ions et on fait passer en retour la solution contenant des polyglycérines, de préférence de la diglycérine, qui quitte l'échangeur d'anions jusqu'à ce que l'on obtienne une teneur en polyglycérine, de préférence une teneur en diglycérine, de 20 % en poids, de préférence jusqu'à ce que l'on obtienne une teneur en polyglycérine, de préférence une teneur en diglycérine, de 15 % en poids, et on l'utilise simultanément pour la préparation de la solution de départ contenant des polyglycérines, de préférence contenant de la diglycérine, à raison de
70 - 40 % en poids, de préférence de
60 - 50 % en poids.

11. Procédé selon une ou plusieurs des revendication 1 à 10, caractérisé en ce que le passage de la solution contenant des polyglycérines, de préférence contenant de la diglycérine a lieu à travers l'échangeur d'ions, de préférence sous une surpression.

12. Procédé selon une ou plusieurs des revendication 1 à 11, caractérisé en ce qu'on fait passer la solution contenant des polyglycérines, de préférence contenant de la diglycérine, sous une pression de
1,1 - 10 bar, de préférence de
2 - 6 bar,
à travers l'échangeur d'ions, c'est-à-dire à travers un ou plusieurs échangeurs de cations et au moins un échangeur d'anions.

13. Procédé selon une ou plusieurs des revendication 1 à 12, caractérisé en ce que la matière faisant office d'échangeur d'ions de l'échangeur de cations et/ou de l'échangeur d'anions est recouverte par une plaque persillée, par une plaque perforée ou par un dispositif installé de façon à pouvoir se déplacer en direction ascendante de l'échangeur d'ions, qui recouvre la matière faisant office d'échangeur et qui permet un passage uniforme de liquide, et/ou par une matière inerte exerçant une pression et/ou par une matière synthétique élastique.

14. Procédé selon une ou plusieurs des revendication 1 à 13, caractérisé en ce que les matières faisant office d'échangeur de cations et les matières faisant office d'échangeur d'anions mises en oeuvre résistent à des températures
allant au-delà de 80°C, de préférence
allant au-delà de 100°C.

15. Procédé selon une ou plusieurs des revendication 1 à 14, caractérisé en ce que la matière fortement acide faisant office d'échangeur de cations et la matière faiblement basique faisant office d'échangeur d'anions possèdent une surface interne (mesurée d'après la méthode BET) de
plus de 25 m²/g, de préférence
de 50 à 100 m²/g.

16. Procédé selon une ou plusieurs des revendication 1 à 15, caractérisé en ce qu'on fait passer de manière avantageuse la solution contenant des polyglycérines, de préférence contenant de la diglycérine, à travers l'échangeur d'ions avec un débit
de 0,5 m/h à 15 m/h, de préférence
de 1 m/h à 5 m/h.
